# EUROPEAN PATENT SPECIFICATION

(11) **EP 4 215 753 B1**
(45) Date of publication and mention of the grant of the patent: **05.11.2025**
(21) Application number: 21869004.8
(22) Date of filing: 13.07.2021
(51) Int. Cl.: F04D 13/06, A61M 60/221, A61M 60/38, F04D 29/22, F04D 29/42, F04D 29/041, A61M 60/109, A61M 60/232, A61M 60/419, A61M 60/824, F04D 29/16, F04D 13/02

(54) **CENTRIFUGAL BLOOD PUMP**
ZENTRIFUGALBLUTPUMPE
POMPE SANGUINE CENTRIFUGE

(30) Priority: 16.09.2020 JP 2020155132
(43) Date of publication of application: 26.07.2023
(73) Proprietor: JMS Co., Ltd., Hiroshima-shi Hiroshima 730-8652 (JP)
(72) Inventor: MIYAMURA, Taiki, Hiroshima-shi, Hiroshima 730-8652 (JP)
(74) Representative: Berggren Oy
(86) International application number: PCT/JP2021/026238
(87) International publication number: WO 2022/059317

(56) References cited:
- WO-A1-2012/115155
- JP-A- 2012 055 485
- JP-A- 2018 061 600
- JP-A- 2018 061 600
- JP-A- H05 212 112
- US-A1- 2013 251 516
- US-A1- 2016 208 804

## Description

### TECHNICAL FIELD

The present invention relates to a centrifugal blood pump.

### BACKGROUND ART

For example, an extracorporeal blood circulation circuit such as an artificial heart-lung circuit is used in heart surgery to temporarily replace a patient's cardiopulmonary function. The extracorporeal blood circulation circuit includes a blood pump configured to cause blood to flow through the circuit. As a blood pump, a centrifugal blood pump is known that applies a centrifugal force to blood to feed the blood by rotating an impeller (a vane wheel) in a pump chamber (a blood containing portion).

In the extracorporeal blood circulation circuit, when blood is circulated using the centrifugal blood pump for a long period, a blood clot may be formed in the centrifugal blood pump. When the blood clot is formed, a flow of blood can grow worse, resulting in deterioration of performance of the centrifugal blood pump.

Conventionally, a structure of a centrifugal blood pump is disclosed in which an impeller axially supported by an upper bearing and a lower bearing is rotatably disposed in a housing (for example, see Patent Document 1). In the housing of the centrifugal blood pump disclosed in Patent Document 1, an inlet port is provided at a top and an outlet port is provided at a side. Blood flowing into the housing from the inlet port passes through the upper bearing of the impeller and is carried away outward in a radial direction by a centrifugal force due to rotation of the impeller. The blood carried away outward in the radial direction is discharged from the outlet port to the outside of the housing.

Patent Document 1: Japanese Unexamined Patent Application, Publication No. 2012-193658

Further relevant prior art can be found in JP 2018 061600 A, US 2016/208804 A1, US 2013/251516 A1, JP 2012 055485 A.

### DISCLOSURE OF THE INVENTION

The invention is defined in the appended claims.

### Problems to be Solved by the Invention

In the centrifugal blood pump disclosed in Patent Document 1, the impeller is axially supported by the upper bearing and the lower bearing, and the blood flowing into the housing from the inlet port stays in the upper bearing, whereby a blood clot may be formed. For this reason, it is desirable to be capable of preventing the blood clot from being formed.

It is an object of the present invention to provide a centrifugal blood pump capable of preventing formation of a blood clot.

### Means for Solving the Problems

The present invention relates to a centrifugal blood pump including: a housing including a blood containing portion that includes an upper surface portion, a lower surface portion, and a side surface portion and contains blood, an inlet port that is provided in communication with the blood containing portion and opens upward, and an outlet port that is provided in communication with the blood containing portion and opens sidewards; an impeller disposed inside the housing and including a base, a top shroud having a blood introduction opening provided spaced above the base and provided at a center in a radial direction, a plurality of guide portions that are provided between the base and the top shroud and guide blood introduced from the blood introduction opening outward in the radial direction, a rotating shaft member having a lower end axially supported by the lower surface portion of the housing and rotatable about a rotation axis, and a first magnetic body disposed below the base; and a magnetic drive unit disposed below the blood containing portion, the magnetic drive unit including a second magnetic body disposed below the first magnetic body and magnetically coupled to the first magnetic body, and configured to drive the impeller rotationally about the rotation axis in a state where the first magnetic body and the second magnetic body are magnetically coupled by a magnetic coupling force to attract each other in an up-down direction, in which, a top surface of the top shroud and a bottom surface of the upper surface portion of the blood containing portion are provided to narrow from an outer side toward an inner side in the radial direction of the top shroud.

Preferably, the top surface of the top shroud is formed in a linear shape that is inclined downward from the inner side toward the outer side in the radial direction, and the bottom surface of the upper surface portion of the blood containing portion is formed in a linear shape that is inclined downward from the inner side toward the outer side in the radial direction.

An angle difference between the top surface of the top shroud and the bottom surface of the upper surface portion of the blood containing portion is set to 2.5° to 4.0°.

Preferably, a bottom surface of the impeller is disposed in parallel with a top surface of a lower surface portion of the blood containing portion.

### Effects of the Invention

According to the present invention, it is possible to provide a centrifugal blood pump capable of preventing a blood clot from being formed.

### BRIEF DESCRIPTION OF THE DRAWINGS

Fig. 1 is a perspective view of a centrifugal blood pump according to an embodiment;
Fig. 2 is a cross-sectional view taken along line A-A in Fig. 1;
Fig. 3 is a perspective view of an impeller of the centrifugal blood pump as viewed from above; and
Fig. 4 is a perspective view of an impeller of the centrifugal blood pump as viewed from below.

### PREFERRED MODE FOR CARRYING OUT THE INVENTION

A centrifugal blood pump 1 according to an embodiment of the present invention will be described below with reference to the drawings. The centrifugal blood pump 1 of the present invention is, for example, a blood pump that is used to an extracorporeal blood circulation circuit such as an artificial heart-lung circuit to circulate blood.

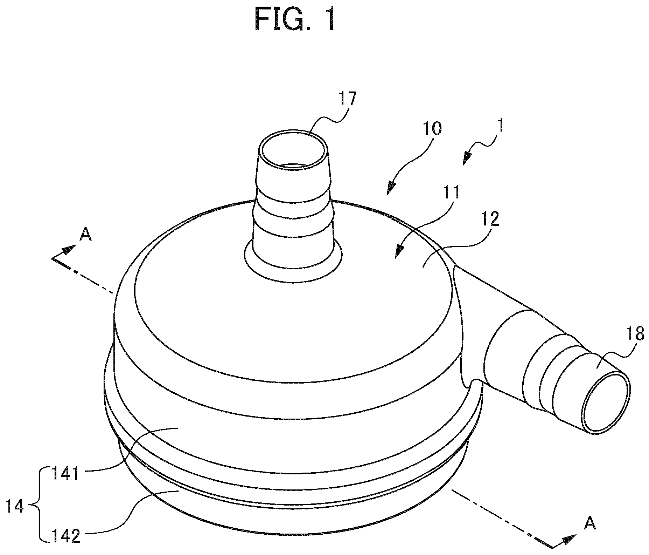

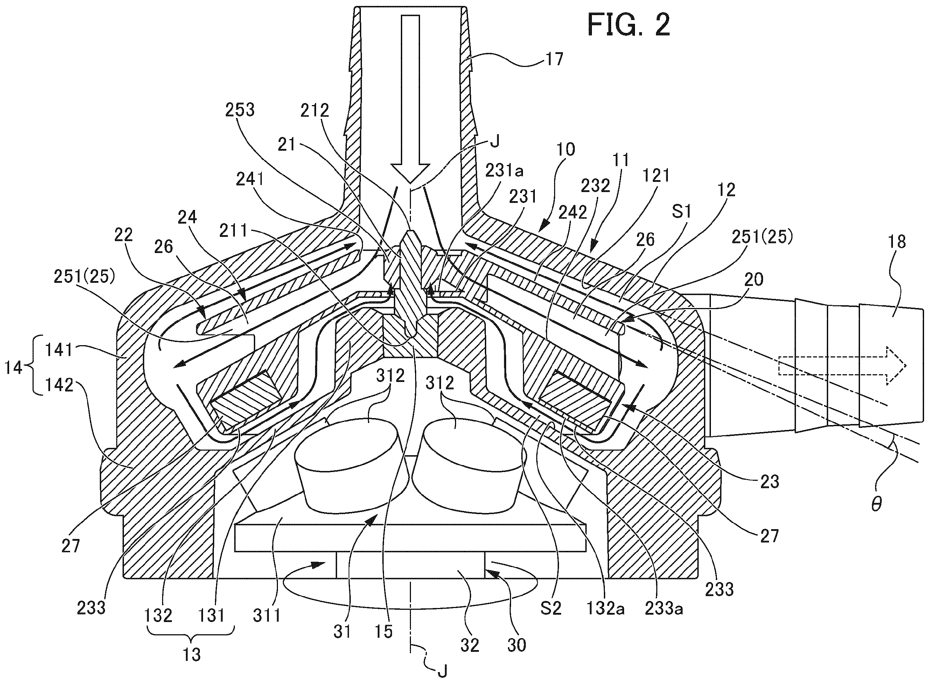

As shown in Figs. 1 and 2, the centrifugal blood pump 1 includes a housing 10, an impeller 20 (a vane wheel) housed inside the housing 10, and a drive device 30 (a magnetic drive unit). The impeller 20 is rotatable about a rotation axis J inside a pump chamber 11 by a driving force of the drive device 30.

The housing 10 includes a pump chamber 11 (blood containing portion), an inlet port 17, and an outlet port 18, as shown in Figs. 1 and 2. The inlet port 17 and the outlet port 18 communicate with the inside of the pump chamber 11.

The pump chamber 11 accommodates the impeller 20 and temporarily contains blood supplied from the inlet port 17. The blood contained in the pump chamber 11 is discharged to the outside through the outlet port 18 by a centrifugal force caused by rotation of the impeller 20. As shown in Fig. 2, the pump chamber 11 includes an upper surface portion 12, a lower surface portion 13, a peripheral wall portion 14 (a side surface portion), and a lower bearing 15.

The upper surface portion 12 is disposed above the pump chamber 11, as shown in Fig. 2. The upper surface portion 12 is formed in an annular plate shape and is gradually inclined downward from an inner side toward an outer side in a radial direction. A bottom surface 121 of the upper surface portion 12 is formed in a linear shape that is inclined downward from an inner side toward an outer side in the radial direction.

The inlet port 17 is connected to a top of the center of the upper surface portion 12. The inlet port 17 is formed in communication with the inside of the pump chamber 11 and opens upward. The inlet port 17 extends upward in the same direction as a rotation axis J of a rotating shaft member 21 from the top of the pump chamber 11.

The lower surface portion 13 is disposed below the upper surface portion 12 with a space therebetween. The lower surface portion 13 includes a central projection 131 formed at a center and protruding upward and an annular inclination part 132 formed in an annular shape outside the central projection 131.

The lower bearing 15 is disposed at a center in the radial direction of the central projection 131. A lower end 211 of the rotating shaft member 21 of the impeller 20, which will be described below, is inserted into the lower bearing 15. The lower bearing 15 axially supports the lower end 211 of the rotating shaft member 21 such that the lower end 211 is rotatable about the rotation axis J.

The annular inclination part 132 is formed in an annular shape that is inclined downward from an inner side toward an outer side in the radial direction. A top surface 132a of the annular inclination part 132 is formed in a linear shape that is inclined downward from an inner side toward an outer side in the radial direction of the lower surface portion 13.

As shown in Fig. 2, the peripheral wall portion 14 forms a peripheral wall of the pump chamber 11, and is formed in a cylindrical shape extending downward from an outer end in the radial direction of the upper surface portion 12. The peripheral wall portion 14 includes an upper peripheral wall portion 141 and a lower peripheral wall portion 142.

The upper peripheral wall portion 141 connects a radial end of the upper surface portion 12 and a circumferential end of the lower surface portion 13 and extends in an up-down direction. An inner surface of the upper peripheral wall portion 141 is formed so as to be recessed in an arc shape outward in the radial direction on an upper side of a longitudinal section, and is formed so as to be inclined downward from an outer side toward an inner side in the radial direction on a lower side of the longitudinal section.

As shown in Fig. 1, the outlet port 18 is connected to an outer surface of the upper peripheral wall portion 141. The outlet port 18 is formed in communication with the inside of the pump chamber 11 and opens sidewards. In the upper peripheral wall portion 141 of the pump chamber 11, the outlet port 18 extends in a horizontal direction along a tangential line of a circle with the rotation axis J of the rotating shaft member 21 as a center.

The lower peripheral wall portion 142 extends downward from a lower end of the upper peripheral wall portion 141. The lower peripheral wall portion 142 is formed in a cylindrical shape that is thicker than the upper peripheral wall portion 141. Inside the lower peripheral wall portion 142, a rotor 31 of the drive device 30, which will be described below, is disposed.

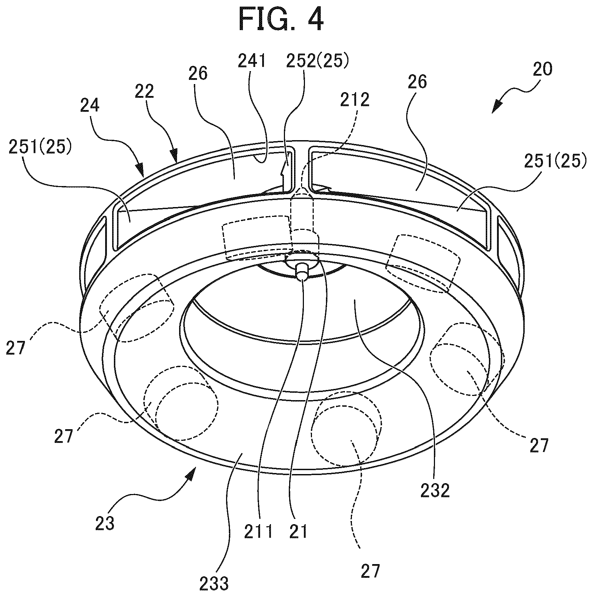

The impeller 20 is disposed inside the pump chamber 11 of the housing 10 so as to be rotatable about the rotation axis J, as shown in Fig. 2. As shown in Figs. 2 to 4, the impeller 20 includes a rotating shaft member 21 rotatable about the rotation axis J, an impeller body 22 coupled to the rotating shaft member 21, and a plurality of impeller-side magnets 27 (first magnetic body). The impeller body 22 includes a base 23, a top shroud 24, a plurality of vanes 25 (guide members) (first vane 251 and second vane 252), and a shaft coupling part 253.

The base 23 is disposed below the impeller body 22. The base 23 includes a horizontal top plate 231, a slope plate 232, and an annular projection 233.

The horizontal top plate 231 is formed in a horizontally annular shape at the center in the radial direction. At the center in the radial direction of the horizontal top plate 231, a through hole 231a is formed so as to penetrate in the up-down direction. The rotating shaft member 21 is disposed to penetrate the through hole 231a. The slope plate 232 is formed in an annular shape that is inclined downward from an outer end in the radial direction of the horizontal top plate 231 toward an outside in the radial direction.

The annular projection 233 extends in an annular shape in a circumferential direction of the base 23 and protrudes downward from a lower part of an outer end in the radial direction of the slope plate 232. The annular projection 233 is formed so as to be inclined downward from the inner side toward the outer side in the radial direction of the base 23.

The bottom surface 233a of the annular projection 233 is formed in a linear shape that is inclined downward from the inner side toward the outer side in the radial direction of the base 23. As shown in Fig. 2, the bottom surface 233a of the annular projection 233 is disposed parallel to the top surface 132a of the annular inclination part 132.

A plurality of impeller-side magnets 27 are disposed inside the annular projection 233. The plurality of impeller-side magnets 27 are disposed at equal intervals in the circumferential direction of the base 23 below the outer side in the radial direction of the base 23. Each of the plurality of impeller-side magnets 27 is disposed in a columnar shape in which an axial length is shorter than a diameter and a top surface and a bottom surface are disposed so as to be inclined downward from the inner side toward the outer side in the radial direction of the base 23.

The top shroud 24 is disposed to face the base 23 at a position spaced upward from the base 23, as shown in Figs. 2 to 4. The top shroud 24 is formed in a plate-like annular shape. The top shroud 24 is inclined downward from then inner side toward the outer side in the radial direction. A top surface 242 of the top shroud 24 is formed in a linear shape that is inclined downward from the inner side toward the outer side in the radial direction.

At a center in the radial direction of the top shroud 24, a circular blood introduction opening 241 is formed to penetrate the top shroud 24 in the up-down direction. A top of the rotating shaft member 21 is disposed to penetrate the blood introduction opening 241, through which the blood supplied from the inlet port 17 to the pump chamber 11 circulates.

An outer diameter of the top shroud 24 is formed to have an outer diameter substantially equal to the outer diameter of the top surface of the base 23, as shown in Fig. 2. A gap S1 between the top surface 242 of the top shroud 24 and the bottom surface 121 of the upper surface portion 12 of the pump chamber 11 is formed to be inclined downward from the inner side toward the outer side in the radial direction of the top shroud 24 and is formed to widen from the inner side toward the outer side in the radial direction. In other words, the gap S1 between the top surface 242 of the top shroud 24 and the bottom surface 121 of the upper surface portion 12 of the pump chamber 11 is formed to narrow from the outer side toward the inner side in the radial direction of the top shroud 24.

An angle difference θ between the top surface 242 of the top shroud 24 and the bottom surface 121 of the upper surface portion 12 of the pump chamber 11 is 2.5° to 4.0°, and more preferably 3.0° to 3.5°. In the present embodiment, the angle difference θ between the top surface 242 of the top shroud 24 and the bottom surface 121 of the upper surface portion 12 of the pump chamber 11 is set to 3.0°. The reason why the angle difference θ between the top surface 242 of the top shroud 24 and the bottom surface 121 of the upper surface portion 12 of the pump chamber 11 is 2.5° to 4.0° will be described below.

The plurality of vanes 25 are disposed between the base 23 and the top shroud 24, as shown in Figs. 2 to 4. Each of the plurality of vanes 25 is formed in a plate shape extending in the up-down direction and in the radial direction so as to connect the base 23 and the top shroud 24. The plurality of vanes 25 guide the blood introduced from the blood introduction opening 241 of the top shroud 24 outward in the radial direction. In the present embodiment, the plurality of vanes 25 include three first vanes 251 and three second vanes 252.

The first vanes 251 and the second vanes 252 are alternately disposed at equal intervals in the circumferential direction of the top shroud 24. Lower end edges of the first vanes 251 and the second vanes 252 are fixed to the top surface of the base 23. Upper end edges of the first vanes 251 and the second vanes 252 are fixed to the bottom surface of the top shroud 24. Thereby, the base 23 and the top shroud 24 are integrated through the first vanes 251 and the second vanes 252.

Outer ends in the radial direction of the first vanes 251 and the second vanes 252 extend to substantially the same position as the outer ends of the base 23 and the top shroud 24 in the radial direction. An inner end in the radial direction of each of the three first vanes 251 is connected to the shaft coupling part 253 disposed at the center in the radial direction. The rotating shaft member 21 extending in the up-down direction is connected to the shaft coupling part 253 connecting the inner ends of each of the three first vanes 251 in a state of penetrating the shaft coupling part 253. An inner end in the radial direction of each of the three second vanes 252 is not connected to the rotating shaft member 21, and extends in the radial direction to substantially the same position as the outer end of the blood introduction opening 241 of the top shroud 24.

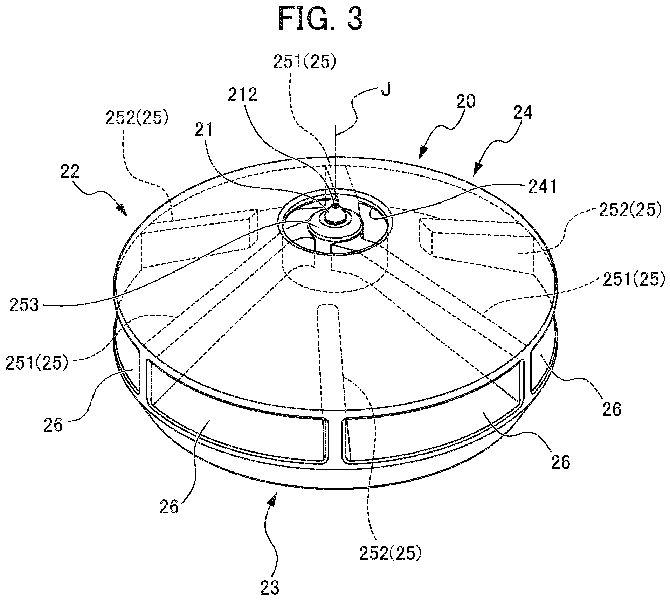

A space between the base 23 and the top shroud 24 which are disposed to face vertically each other are divided into six by the first vane 251 and the second vane 252 adjacent to each other in the circumferential direction, as shown in Fig. 3. Thereby, six guide paths 26 (guide portions) are formed between the base 23 and the top shroud 24 26.

The six guide paths 26 radially extends in the radial direction from the center of the impeller 20. At the center in the radial direction, the top side of each of the guide paths 26 communicates with the blood introduction opening 241 formed at the center in the radial direction of the top shroud 24 and the bottom side thereof communicates with the through hole 231a formed at the center in the radial direction of the base 23. Each of the guide paths 26 opens outward in the radial direction at the outer end in the radial direction of the impeller 20. Each of the guide paths 26 guides the blood introduced between the base 23 and the top shroud 24 from the blood introduction opening 241 outward in the radial direction.

The rotating shaft member 21 is disposed to penetrate the central part in the radial direction of the top shroud 24 in the up-down direction and extends in the up-down direction, as shown in Fig. 2. The rotating shaft member 21 is coupled to the shaft coupling part 253 while penetrating the shaft coupling part 253 connecting the inner ends of the three first vanes 251.

The lower end 211 of the rotating shaft member 21 is axially supported by the lower bearing 15 disposed on the lower surface portion 13 of the housing 10. The lower bearing 15 is provided in the center of the lower surface portion 13 of the pump chamber 11.

An upper end 212 of the rotating shaft member 21 is a free end which is not supported by a bearing, as shown in Fig. 2. In other words, the centrifugal blood pump 1 of the present embodiment has a so-called mono-pivot bearing structure in which the impeller 20 is supported only at one point of the lower bearing 15. In the present embodiment, since the upper end 212 of the rotating shaft member 21 is not supported by a bearing, the blood is less likely to stay at the upper end 212 of the rotating shaft member 21, and thus stagnation of the blood is reduced. Therefore, a blood clot is hardly formed in the vicinity of the upper end 212.

The drive device 30 is disposed below the housing 10. The drive device 30 includes a rotor 31 and a drive shaft 32 capable of rotating the rotor 31 about the rotation axis J. The drive shaft 32 is extendable from a bottom surface of the rotor 31. The drive shaft 32 is coupled to a rotary drive source such as a motor and is driven to rotate. The rotor 31 includes a truncated cone 311 and a plurality of drive-side magnets 312 (second magnetic body) disposed on a top surface of the truncated cone 311.

Below the housing 10, the plurality of drive-side magnets 312 are disposed below the impeller-side magnets 27 to face the plurality of impeller-side magnets 27 of the impeller 20 accommodated inside the housing 10. The plurality of drive-side magnets 312 are disposed with the same number as the impeller-side magnets 27 and are disposed at equal intervals in the circumferential direction of the truncated cone 311. Each of the plurality of drive-side magnets 312 is formed in a columnar shape in which an axial length is shorter than a diameter and a top surface and a bottom surface are disposed so as to be inclined downward from an inner side toward an outer side in the radial direction of the truncated cone 311.

In a case of causing the rotor 31 to rotate about the rotation axis J, the drive device 30 rotates and drives the impeller 20 about the rotation axis J in a state in which the impeller-side magnets 27 and the drive-side magnets 312 are magnetically coupled by a magnetic coupling force acting to attract each other in the up-down direction. For example, the magnetic coupling force between the impeller-side magnets 27 and the drive-side magnets 312 is 12 N.

An operation of the centrifugal blood pump 1 described above will be described. When the rotor 31 of the drive device 30 rotates about the rotation axis J, the impeller 20 rotates about rotation axis J inside the housing 10 in a state in which the drive-side magnets 312 and the impeller-side magnets 27 are magnetically coupled by the magnetic coupling force acting to attract each other in the up-down direction. Thereby, the impeller 20 stably rotates in the state in which the impeller-side magnets 27 and the drive-side magnets 312 are magnetically coupled by the magnetic coupling force.

In this state, blood is supplied from the inlet port 17 to the inside of the pump chamber 11. The blood supplied from the inlet port 17 is introduced into the six guide paths 26 of the impeller 20 through the blood introduction opening 241 formed at the center in the radial direction of the top shroud 24.

The blood introduced into the six guide paths 26 moves from the inner side to the outer side in the radial direction inside the guide paths 26 by receiving the centrifugal force due to the rotation of the impeller 20, flows out from the outer end in the radial direction of each of the guide paths 26 into the pump chamber 11, and is discharged to the outside through the outlet port 18.

In addition, the blood flowing out from the outer end in the radial direction of each of the guide paths 26 into the pump chamber 11 moves toward the through hole 231a of the base 23 through a gap S2 between the bottom surface 233a of the annular projection 233 of the base 23 and the top surface 132a of the lower surface portion 13 of the pump chamber 11. The blood moved toward the through hole 231a through the gap S2 below the base 23 is introduced into the guide paths 26 through the through hole 231a of the base 23. For this reason, a flow of blood (a so-called secondary flow) occurs that moves under the base 23 and returns to the through hole 231a of the base 23. Thereby, a force is generated that pushes up the impeller 20.

Further, the blood flowing out from the outer end in the radial direction of the guide path 26 into the pump chamber 11 moves toward the blood introduction opening 241 through the gap S1 between the top surface 242 of the top shroud 24 and the bottom surface 121 of the upper surface portion 12 of the pump chamber 11. The blood moved toward the blood introduction opening 241 through the gap S1 above the top shroud 24 is introduced into the guide path 26 through the blood introduction opening 241 of the top shroud 24.

Here, the gap S1 is formed so as to narrow from the outer side toward the inner side in the radial direction of the top shroud 24. An angle difference θ is provided between the top surface 242 of the top shroud 24 and the bottom surface 121 of the upper surface portion 12 of the pump chamber 11. Therefore, a pressure is generated by the blood passing through the gap S1 formed between the top surface 242 of the top shroud 24 and the bottom surface 121 of the upper surface portion 12 of the pump chamber 11, thereby pushing the top shroud 24 downward from the above. The pressure generated by the blood passing through the gap S1 presses the impeller 20 downward and prevent the impeller 20 from floating.

A description will be given with respect to the angle difference θ between the top surface 242 of the top shroud 24 and the bottom surface 121 of the upper surface portion 12 of the pump chamber 11. The angle difference θ between the top surface 242 of the top shroud 24 and the bottom surface 121 of the upper surface portion 12 of the pump chamber 11 will be described from the viewpoint of preventing floating of the impeller 20 and from the viewpoint of resistance to hemolysis.

The rotating shaft member 21 of the impeller 20 is configured in which the upper end 212 is not axially supported and the lower end 211 is axially supported. Thereby, the blood is less likely to stay at the upper end 212 of the rotating shaft member 21, and a blood clot is hardly formed in the vicinity of the upper end 212. The drive device 30 rotates, and thus the impeller 20 rotates about rotation axis J in a state in which the drive-side magnets 312 and the impeller-side magnets 27 are magnetically coupled by the magnetic coupling force acting to attract each other in the up-down direction.

On the other hand, the impeller 20 is configured in which the lower end 211 of the rotating shaft member 21 is axially supported, but the upper end 212 is not axially supported. In addition, the blood flowing through the gap S2 between the bottom surface 233a of the annular projection 233 of the base 23 and the top surface 132a of the lower surface portion 13 of the pump chamber 11 pushes up the impeller 20. For this reason, even when the impeller-side magnet 27 and the drive-side magnet 312 are magnetically coupled by the magnetic coupling force, the blood flowing through the gap S2 between the bottom surface 233a of the annular projection 233 of the base 23 and the top surface 132a of the lower surface portion 13 of the pump chamber 11 pushes up the impeller 20, whereby the impeller 20 may float and fall off the lower bearing 15.

On the other hand, according to the present invention, since the angle difference θ is provided between the top surface 242 of the top shroud 24 and the bottom surface 121 of the upper surface portion 12 of the pump chamber 11, a clearance of the gap S1 is formed to be smaller from the outer side toward the inner side in the radial direction of the top shroud 24. Thereby, the impeller 20 can be pushed down from above and the impeller 20 can be prevented from floating by the pressure of the blood flowing through the gap S1 between the top surface 242 of the top shroud 24 and the bottom surface 121 of the upper surface portion 12 of the pump chamber 11.

Here, when the angle difference θ between the top surface 242 of the top shroud 24 and the bottom surface 121 of the upper surface portion 12 of the pump chamber 11 is made larger, a shearing force acting on the blood increases. When the shearing force acting on the blood increases, blood cells may be destroyed (hemolysis) and resistance to hemolysis may not be ensured. Therefore, it is necessary to ensure the resistance to hemolysis by setting the angle difference θ between the top surface 242 of the top shroud 24 and the bottom surface 121 of the upper surface portion 12 of the pump chamber 11 to an angle difference such an extent that the shearing force does not become too large.

Therefore, from the viewpoint of preventing the floating of the impeller 20 and ensuring the resistance to hemolysis, an experiment has been conducted by changing the angle difference θ between the top surface 242 of the top shroud 24 and the bottom surface 121 of the upper surface portion 12 of the pump chamber 11, and based on the result of experiment, the angle difference θ is set such that the floating of the impeller 20 can be prevented and the resistance to hemolysis can be ensured.

In the experiment in which the angle difference θ is changed, the criteria for determining whether the floating of the impeller 20 can be prevented are set as follows. In the present embodiment, the impeller 20 rotates in a state of being magnetically coupled to the rotor 31 of the drive device 30 with a magnetic coupling force of 12 N that acts to attract each other in the up-down direction. In this case, in order to prevent the floating force of the impeller 20 and normally rotate the impeller 20, when a safety factor is taken into account after consideration of a force for pushing up the impeller 20 caused by the secondary flow of the blood and the magnetic coupling force acting on the impeller 20 and the rotor 31 of the drive device 30 to attract each other in the up-down direction, it has been seen by experiment that the maximum floating force of the impeller 20 is preferably 8 N or less. For this reason, as a criterion for the floating force of the impeller 20, when the angle difference θ between the top surface 242 of the top shroud 24 and the bottom surface 121 of the upper surface portion 12 of the pump chamber 11 is changed, the determination is made as "satisfactory" (indicated by circle symbol (o)) when the maximum floating force of the impeller 20 is 8 N or less.

In the experiment in which the angle difference θ is changed, the criteria for determining the resistance to hemolysis are set as follows. When the shearing force is 900 Pa or less, it has been seen by the experiment that the resistance to hemolysis is ensured. For this reason, as a criterion for determining the resistance to hemolysis, when the angle difference θ between the top surface 242 of the top shroud 24 and the bottom surface 121 of the upper surface portion 12 of the pump chamber 11 is changed, the determination is made as "satisfactory" (indicated by circle symbol (o)) when the shearing force is 900 Pa or less.

Results of experiment is indicated in Table 1 in which the maximum floating force of the impeller 20 and the maximum shearing force of the blood are obtained when the angle difference θ between the top surface 242 of the top shroud 24 and the bottom surface 121 of the upper surface portion 12 of the pump chamber 11 is changed.

**[Table 1]**

| Angle difference *θ* (°) | Floating force of impeller | | Resistance to hemolysis | | Comprehensive determination |
|---|---|---|---|---|---|
| | Maximum floating force of impeller (N) | Results of determination "Satisfactory" (indicated by circle symbol (o)) in case of 8 N or less) | Maximum shearing force (Pa) | Results of determination ("Satisfactory" (indicated by circle symbol (o)) in case of 900 Pa or less) | |
| 2.0 | 8.4 | x | 853 | o | △ |
| 2.5 | 7.9 | o | 882 | o | ⊙ |
| 3.0 | 7.5 | o | 757 | o | ⊙ |
| 3.5 | 7.5 | o | 782 | o | ⊙ |
| 4.0 | 7.7 | o | 883 | o | ⊙ |
| 5.0 | 8.1 | x | 1016 | x | x |

In the determination results, the floating force of the impeller 20 was evaluated as "satisfactory" (indicated by circle symbol (o)) when the maximum floating force of the impeller 20 is 8 N or less, and the floating force of the impeller 20 was evaluated as "poor" (indicated by cross symbol (x)) when the maximum floating force of the impeller 20 is more than 8 N. The resistance to hemolysis was evaluated as "satisfactory" (indicated by circle symbol (o)) when the shearing force is 900 Pa or less, and the resistance to hemolysis was evaluated as "poor" (indicated by cross symbol (x)) when the shearing force is more than 900 Pa. When both the floating force of the impeller 20 and the resistance to hemolysis satisfy the determination criteria (when both are evaluated as "satisfactory" (indicated by circle symbol (o))), it was evaluated as "excellent" (indicated by concentric circle symbol (⊙) in comprehensive determination.

According to the results of experiment indicated in Table 1, when the angle difference θ between the top surface 242 of the top shroud 24 and the bottom surface 121 of the upper surface portion 12 of the pump chamber 11 is 2.5° to 4.0°, both the floating force of the impeller 20 and the resistance to hemolysis are evaluated as "satisfactory" (indicated by circle symbol (o)), which satisfy the determination criteria, and thus it is evaluated as "excellent" (indicated by concentric circle symbol (⊙) in comprehensive determination. Therefore, the angle difference θ between the top surface 242 of the top shroud 24 and the bottom surface 121 of the upper surface portion 12 of the pump chamber 11 is 2.5° to 4.0°. Furthermore, the angle difference θ between the top surface 242 of the top shroud 24 and the bottom surface 121 of the upper surface portion 12 of the pump chamber 11 is more preferably 3.0° to 3.5°, which is a median value in the range of 2.5° to 4.0°. In the present embodiment, the angle difference θ between the top surface 242 of the top shroud 24 and the bottom surface 121 of the upper surface portion 12 of the pump chamber 11 is set to 3.0°.

According to the centrifugal blood pump 1 of the present embodiment described above, the following effects are obtained. The centrifugal blood pump 1 includes: the housing 10; the impeller 20 disposed inside the housing 10 and including the base 23, the top shroud 24 having the blood introduction opening 241 formed at the center in the radial direction, the plurality of guide paths 26 that guides the blood introduced from the blood introduction opening 241 outward in the radial direction, the rotating shaft member 21 in which the lower end 211 is axially supported by the lower surface portion 13 of the housing 10 and is rotatable about the rotation axis J, and the plurality of impeller-side magnets 27 disposed below the base 23; and the magnetic drive unit 30 that drives the impeller 20 rotationally about the rotation axis J in a state where the impeller-side magnet 27 and the drive-side magnet 312 are magnetically coupled by the magnetic coupling force to attract each other in the up-down direction, and the top surface 242 of the top shroud 24 and the bottom surface 121 of the upper surface portion 12 of the pump chamber 11 are formed to narrow from the outer side toward the inner side in the radial direction of the top shroud 24.

Thereby, since the upper end 212 of the rotating shaft member 21 is not supported by a bearing, a configuration is provided in which a blood clot is hardly formed, and thus even when the upper end 212 of the rotating shaft member 21 is not supported by the bearing, the top surface 242 of the top shroud 24 and the bottom surface 121 of the upper surface portion 12 of the pump chamber 11 are formed to narrow from the outer side toward the inner side in the radial direction of the top shroud 24, whereby the pressure of the blood can be applied from the upper side to the lower side of the impeller 20 to push the impeller 20 downward. As a result, the blood clot can be prevented from being formed, and the impeller 20 can be stably rotated by preventing the floating of the impeller 20.

In addition, the top surface 242 of the top shroud 24 is formed in a linear shape that is inclined downward from the inner side toward the outer side in the radial direction, and the bottom surface 121 of the upper surface portion 12 of the pump chamber 11 is formed in a linear shape that is inclined downward from the inner side toward the outer side in the radial direction. Thus, since the gap S1 between the top surface 242 of the top shroud 24 and the bottom surface 121 of the upper surface portion 12 of the pump chamber 11 is gradually narrowed, the pressure of the blood can be stably generated to push the impeller 20 downward, and thus the impeller 20 can be rotated more stably.

In addition, the angle difference θ between the top surface 242 of the top shroud 24 and the bottom surface 121 of the upper surface portion 12 of the pump chamber 11 is set to 2.5° to 4.0°. Thus, it is possible to prevent the floating force of the impeller 20 and to ensure the resistance to hemolysis.

In addition, the bottom surface 233a of the annular projection 233 of the impeller 20 is disposed in parallel with the top surface 132a of the annular inclination part 132 of the lower surface portion 13 of the pump chamber 11. Thus, since the pressure of the blood circulating through the gap S2 is constant, it is possible to stably rotate the impeller 20.

In the above-described embodiment, the rotating shaft member 21 is disposed to penetrate the central part in the radial direction of the top shroud 24 in the up-down direction, but the present invention is not limited thereto. Since the upper end of the rotating shaft member 21 is not axially supported, the upper end side of the rotating shaft member 21 may not have to penetrate the top shroud 24.

### EXPLANATION OF REFERENCE NUMERALS

1 centrifugal blood pump
10 housing
11 pump chamber (blood containing portion)
12 upper surface portion
13 lower surface portion
14 peripheral wall portion (side surface portion)
17 inlet port
18 outlet port
20 impeller
21 rotating shaft member
23 base
24 top shroud
25 vane (guide member)
26 guide path (guide portion)
27 impeller-side magnet (first magnetic body)
30 drive device (magnetic drive unit)
121 bottom surface of upper surface portion of pump chamber (blood containing portion)
132a top surface of annular inclination part (top surface of lower surface portion)
211 lower end
233a bottom surface of annular projection (bottom surface of impeller)
241 blood introduction opening
242 top surface of top shroud
312 drive-side magnet (second magnetic body)
J rotation axis
θ angle difference

## Claims

1. A centrifugal blood pump (1) comprising: a housing (10) including a blood containing portion (11) that includes an upper surface portion (12), a side surface portion (14), and a lower surface portion (13) and is configured to contain blood, an inlet port (17) that is provided in communication with the blood containing portion (11) and opens upward, and an outlet port (18) that is provided in communication with the blood containing portion (11) and opens sidewards;
an impeller (20) disposed inside the housing (10) and including a base (23), a top shroud (24) having a blood introduction opening (241) provided spaced above the base (23) and provided at a center in a radial direction, a plurality of guide portions (26) that are provided between the base (23) and the top shroud (24) and guide blood introduced from the blood introduction opening (241) outward in the radial direction, a rotating shaft member (21) having a lower end (211) axially supported by the lower surface portion (13) of the housing (10) and rotatable about a rotation axis (J), and a first magnetic body (27) disposed below the base (23); and
a magnetic drive unit (30) disposed below the blood containing portion (11), the magnetic drive unit (30) including a second magnetic body (312) disposed below the first magnetic body (27) and magnetically coupled to the first magnetic body (27), and configured to drive the impeller (20) rotationally about the rotation axis in a state where the first magnetic body (27) and the second magnetic body (312) are magnetically coupled by a magnetic coupling force to attract each other in an up-down direction,
**characterized in that**
a top surface (242) of the top shroud (24) and a bottom surface of the upper surface portion (12) of the blood containing portion (11) are provided to narrow from an outer side toward an inner side in the radial direction of the top shroud (24), such that a gap (S1) between the top surface (242) of the top shroud (24) and the bottom surface of the upper surface portion (12) of the blood containing portion (11) is formed to narrow from the outer side toward the inner side in the radial direction of the top shroud (24), and wherein an angle difference between the top surface (242) of the top shroud (24) and the bottom surface of the upper surface portion (12) of the blood containing portion (11) is set to 2.5° to 4.0°.

2. The centrifugal blood pump (1) according to claim 1, wherein the top surface (242) of the top shroud (24) is provided in a linear shape that is inclined downward from the inner side toward the outer side in the radial direction, and
the bottom surface (121) of the upper surface portion (12) of the blood containing portion (11) is provided in a linear shape that is inclined downward from the inner side toward the outer side in the radial direction.

3. The centrifugal blood pump (1) according to claim 1 to 2,
wherein a bottom surface (23a) of the impeller (20) is disposed in parallel with a top surface (132a) of a lower surface portion (13) of the blood containing portion (11).

## Patentansprüche

1. Zentrifugalblutpumpe (1), umfassend: ein Gehäuse (10) einschließlich eines bluthaltigen Abschnitts (11), der einen oberen Flächenabschnitt (12), einen seitlichen Flächenabschnitt (14) und einen unteren Flächenabschnitt (13) aufweist und konfiguriert ist, um Blut zu enthalten, einen Einlassanschluss (17), der in Verbindung mit dem bluthaltigen Abschnitt (11) bereitgestellt ist und sich nach oben öffnet, und einen Auslassanschluss (18), der in Verbindung mit dem bluthaltigen Abschnitt (11) bereitgestellt ist und sich seitlich öffnet;
ein Laufrad (20), das innerhalb des Gehäuses (10) angeordnet ist und eine Basis (23) enthält, eine obere Abdeckung (24) mit einer Bluteinführungsöffnung (241), die oberhalb der Basis (23) beabstandet und in radialer Richtung mittig bereitgestellt ist, eine Vielzahl von Führungsabschnitten (26), die zwischen der Basis (23) und der oberen Abdeckung (24) bereitgestellt sind und das aus der Bluteinführungsöffnung (241) eingeführte Blut in radialer Richtung nach außen leiten, ein rotierendes Wellenelement (21) mit einem unteren Ende (211), das axial von dem unteren Flächenabschnitt (13) des Gehäuses (10) gestützt wird und um eine Rotationsachse (J) drehbar ist, und einen ersten Magnetkörper (27), der unterhalb der Basis (23) angeordnet ist; und
eine magnetische Antriebseinheit (30), die nach dem bluthaltigen Abschnitt (11) angeordnet ist, wobei die magnetische Antriebseinheit (30) einen zweiten Magnetkörper (312) umfasst, der nach dem ersten Magnetkörper (27) angeordnet und magnetisch mit dem ersten Magnetkörper (27) gekoppelt ist und konfiguriert ist, um das Laufrad (20) um die Rotationsachse in einem Status anzutreiben, in dem der erste Magnetkörper (27) und der zweite Magnetkörper (312) durch eine magnetische Kopplungskraft magnetisch gekoppelt sind, um sich in einer Aufwärts-Abwärts-Richtung anzuziehen,
**dadurch gekennzeichnet, dass**
eine obere Fläche (242) der oberen Abdeckung (24) und eine untere Fläche des oberen Flächenabschnitts (12) des bluthaltigen Abschnitts (11) bereitgestellt sind, um sich von einer Außenseite zu einer Innenseite in radialer Richtung der oberen Abdeckung (24) zu verengen, derart, dass eine Lücke (S1) zwischen der oberen Fläche (242) der oberen Abdeckung (24) und der unteren Fläche des oberen Flächenabschnitts (12) des bluthaltigen Abschnitts (11) ausgebildet wird, die sich von der Außenseite zur Innenseite in radialer Richtung der oberen Abdeckung (24) verengt und wobei ein Winkelunterschied zwischen der oberen Fläche (242) der oberen Abdeckung (24) und der unteren Fläche des oberen Flächenabschnitts (12) des bluthaltigen Abschnitts (11) auf 2,5° bis 4,0° eingestellt ist.

2. Zentrifugalblutpumpe (1) nach Anspruch 1, wobei die obere Fläche (242) der oberen Abdeckung (24) in einer linearen Form bereitgestellt ist, die von der Innenseite zur Außenseite in radialer Richtung nach unten geneigt ist, und
die untere Fläche (121) des oberen Flächenabschnitts (12) des bluthaltigen Abschnitts (11) in einer linearen Form bereitgestellt ist, die von der Innenseite zur Außenseite in radialer Richtung nach unten geneigt ist.

3. Zentrifugalblutpumpe (1) nach Anspruch 1 bis 2, wobei eine untere Fläche (23a) des Laufrads (20) parallel zu einer oberen Fläche (132a) eines unteren Flächenabschnitts (13) des bluthaltigen Abschnitts (11) angeordnet ist.

## Revendications

1. Pompe à sang centrifuge (1) comprenant : un boîtier (10) comportant une partie contenant du sang (11) qui comporte une partie de surface supérieure (12), une partie de surface latérale (14) et une partie de surface inférieure (13) et qui est conçue pour contenir du sang, un orifice d'entrée (17) qui est prévu en communication avec la partie contenant du sang (11) et s'ouvre vers le haut, et un orifice de sortie (18) qui est prévu en communication avec la partie contenant du sang (11) et s'ouvre latéralement ;
un rotor (20) disposé à l'intérieur du boîtier (10) et comportant une base (23), un capot supérieur (24) ayant une ouverture d'introduction de sang (241) prévue à distance au-dessus de la base (23) et prévue au niveau d'un centre dans un sens radial, une pluralité de parties de guidage (26) qui sont prévues entre la base (23) et le capot supérieur (24) et guident le sang introduit à partir de l'ouverture d'introduction de sang (241) vers l'extérieur dans le sens radial, un élément d'arbre rotatif (21) ayant une extrémité inférieure (211) supportée axialement par la partie de surface inférieure (13) du boîtier (10) et pouvant tourner autour d'un axe de rotation (J), et un premier corps magnétique (27) disposé en dessous de la base (23) ; et une unité d'entraînement magnétique (30) disposée en dessous de la partie contenant du sang (11), l'unité d'entraînement magnétique (30) comportant un second corps magnétique (312) disposé en dessous du premier corps magnétique (27) et accouplé magnétiquement au premier corps magnétique (27), et conçue pour entraîner le rotor (20) en rotation autour de l'axe de rotation dans un état où le premier corps magnétique (27) et le second corps magnétique (312) sont accouplés magnétiquement par une force d'accouplement magnétique pour s'attirer l'un l'autre dans un sens de haut en bas,
**caractérisée en ce que**
une surface supérieure (242) du capot supérieur (24) et une surface inférieure de la partie de surface supérieure (12) de la partie contenant du sang (11) sont prévues pour se rétrécir à partir d'un côté externe vers un côté interne dans le sens radial du capot supérieur (24), de sorte qu'un espace (SI) entre la surface supérieure (242) du capot supérieur (24) et la surface inférieure de la partie de surface supérieure (12) de la partie contenant du sang (11) est formé pour se rétrécir à partir du côté externe vers le côté interne dans le sens radial du capot supérieur (24), et dans laquelle une différence d'angle entre la surface supérieure (242) du capot supérieur (24) et la surface inférieure de la partie de surface supérieure (12) de la partie contenant du sang (11) est fixée entre 2,5° et 4,0°.

2. Pompe à sang centrifuge (1) selon la revendication 1, dans laquelle la surface supérieure (242) du capot supérieur (24) est prévue sous une forme linéaire qui est inclinée vers le bas à partir du côté interne vers le côté externe dans le sens radial, et
la surface inférieure (121) de la partie de surface supérieure (12) de la partie contenant du sang (11) est prévue sous une forme linéaire qui est inclinée vers le bas à partir du côté interne vers le côté externe dans le sens radial.

3. Pompe à sang centrifuge (1) selon les revendications 1 à 2, dans laquelle une surface inférieure (23a) du rotor (20) est disposée en parallèle avec une surface supérieure (132a) d'une partie de surface inférieure (13) de la partie contenant du sang (11).
